# DEMANDE DE BREVET EUROPEEN

(11) **EP 1 088 548 A2**
(43) Date de publication de la demande: **04.04.2001**
(21) Numéro de dépôt: 00402460.0
(22) Date de dépôt: 07.09.2000
(51) Int. Cl.: A61K 7/48

(54) **Utilisation de l'alvérine pour diminuer les rides**

(30) Priorité: 21.09.1999 FR 9911772
(71) Demandeur: L'OREAL, 75008 Paris (FR)
(72) Inventeur: Liviero, Christel, 75008 Paris (FR); Breton, Lionel, 78000 Versailles (FR); Pineau, Nathalie, Résidence des jardins du Clain, 86000 Poitiers (FR)
(74) Mandataire: Galup, Cédric Olivier Nicolas

(57) **Abrégé**

La présente invention se rapporte à l'utilisation d'au moins une quantité efficace d'Alvérine ou de l'un de ses sels, dans une composition ou pour la préparation d'une composition, l'Alvérine ou son sel ou la composition étant destinées à relâcher et/ou relaxer le tissu cutané et/ou sous-cutané, notamment en vue de diminuer les rides et les ridules de la peau.

## Description

La présente invention se rapporte à l'utilisation d'au moins une quantité efficace d'Alvérine ou de l'un de ses sels dans une composition ou pour la préparation d'une composition, l'Alvérine ou son sel ou la composition étant destinées à relâcher et/ou relaxer le tissu cutané et/ou sous-cutané, notamment en vue de diminuer les rides et les ridules de la peau.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans rides, marques d'une peau jeune, d'autant plus que l'aspect physique induit sur le psychisme et/ou sur le moral. Or, il est important de se sentir physiquement et moralement jeune.

Jusqu'à présent, on réduisait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Mais, à ce jour, on ne sait pas agir sur les rides en intervenant sur les éléments musculaires présents dans la peau. C'est dans cette voie que se place la présente invention.

Il est connu que les muscles peauciers du visage sont sous le contrôle des afférences nerveuses motrices du nerf facial et que, par ailleurs, les cloisons inter lobulaires de l'hypoderme contiennent en leur sein des fibres qui constituent un tissu musculaire strié (panniculus carnosus). D'autre part, il est également connu qu'une sous-population de fibroblastes du derme, que l'on appelle myofibroblastes, présente des caractéristiques communes avec le tissu musculaire.

La demanderesse a notamment observé, dans certaines situations pathologiques et thérapeutiques, le rôle joué sur les rides du visage par les nerfs contrôlant l'ensemble de ce tissu musculaire. Ainsi, dans les atteintes du nerf facial, dans lesquelles la transmission de l'influx nerveux est interrompue et/ou amoindrie, on assiste dans le territoire d'innervation à une paralysie des muscles du visage. Cette paralysie faciale se traduit, entre autres signes cliniques, par une atténuation, voire une disparition des rides.

A l'inverse, dans les états d'hyper contraction musculaire de la face, la demanderesse a constaté une accentuation des rides du visage. De plus, elle a également observé une accentuation des rides du visage dans les états d'hypertonie musculaire de la maladie de Parkinson et des effets secondaires induits par les neuroleptiques.

Par ailleurs, il a été montré que la toxine botulique, utilisée à l'origine pour diminuer les spasmes, pouvait agir sur les états de spasticité musculaire (voir A. Blitzer et al., Arch. Otolaryngol. Head Neck Surg., 1993, 119, pages 1018 à 1022) et sur les rides de la glabelle qui sont les rides inter sourcilières (voir J.D. Carruters et al., J. Dermatol. Surg. Oncol., 1992, 18, pages 17 à 21). Cette toxine botulique est d'ailleurs classiquement utilisée en chirurgie plastique dans le traitement des rides. En conséquence, il est possible d'agir par une action pharmacologique sur la composante neuromusculaire des rides.

Après de nombreux tests, la demanderesse a pu déterminer que les fibres musculaires contractiles, en particulier des fibres musculaires striées, qui se trouvent sous le contrôle direct de l'influx neuro-musculaire, jouaient un rôle essentiel dans la formation des rides et que la modulation de l'influx neuromusculaire atténuait non seulement les rides mais également les ridules et avait aussi un effet de "lissage" sur le microrelief cutané.

Physiologiquement, l'activité des muscles striés (à l'exception du cardiomyocyte) est déclenchée par le système nerveux, celle des muscles lisses est spontanée et modulée par divers stimuli de type hormonaux ou nerveux.

Les cellules à l'origine des contractions spontanées, sont des cellules "pacemakers" : la propagation de l'onde d'excitation électrique se fait de proche en proche au niveau de zone de moindre résistance électrique des membranes cellulaires.

En dehors de l'activité spontanée, l'excitation des fibres musculaires lisses peut être déclenchée par différents stimuli : étirement mécanique, présence de neurotransmetteurs ou d'hormones intestinales venant agir spécifiquement au niveau de certains récepteurs cellulaires, certains agents ayant un effet agoniste ou antagoniste sur ces même récepteurs.

Quelque soit le type de fibre (lisse ou striée), le messager final commun à l'origine de la contraction est l'ion calcium (Ca²⁺).
Cependant, l'intervention de ce cation dans le mécanisme contractile est tout à fait différente suivant le type de fibre (lisse ou striée).

Les travaux du groupe de Ebashi ont permis de montrer que, dans les muscles lisses (contrairement aux muscles striés) le glissement des molécules d'actine et de myosine ne se produit pas après la levée par le Ca²⁺ de l'inhibition exercée par la troponine. Le cation exerce un effet activateur direct sur la liaison actine-myosine en association avec une protéine appelée leiotonine C. L'action conjointe du Ca²⁺ et de la leiotonine C est responsable de la phosphorylation des chaînes légères de la myosine qui provoque la contraction.

De plus, la relaxation des muscles lisses à la différence du muscle strié est très active. Elle est dépendante de médiateurs hormonaux et nerveux.

La mise en évidence de ces activités physiologiques est à l'origine du contrôle de la motricité digestive par des agents pharmacologiques.
Ainsi, l'Alvérine ou di(phénylpropyl)éthylamine, encore nommée Spasmavérine ou Diproline, est connue comme relaxant du muscle lisse digestif.

La demanderesse a maintenant découvert que l'effet relaxant de l'Alvérine n'est pas spécifique du muscle lisse.
L'Alvérine peut induire un effet décontracturant et/ ou relaxant sur le muscle strié et donc, par voie de conséquence, constituer un actif intéressant pour la correction des rides et /ou des ridules et pour favoriser le lissage de la peau.

A la connaissance de la demanderesse rien dans l'art antérieur ne décrit ni ne suggère une telle propriété.

La présente invention se rapporte donc à l'utilisation, dans une composition ou pour la préparation d'une composition, d'au moins une quantité efficace d'Alvérine ou de l'un de ses sels, l'Alvérine ou son sel ou la composition étant destinées à relaxer et/ou relâcher le tissu cutané et/ou sous-cutané.

Par sels d'alvérine on entend selon l'invention les sels organiques ou inorganiques d'alvérine.

Comme sels organiques utilisables selon l'invention on peut citer le gluconate d'alvérine ou l'acétate d'alvérine ou le citrate d'alvérine ou l'oléate d'alvérine ou l'oxalate d'alvérine.

Comme sels inorganiques d'alvérine on peut citer les sels minéraux comme le chlorure d'alvérine ou le borate d'alvérine ou le nitrate d'alvérine ou le phosphate d'alvérine ou le sulfate d'alvérine ou le carbonate d'alvérine.

On comprend ainsi que dans le texte, sous réserve d'indication contraire, l'emploi du terme alvérine doit être compris comme signifiant aussi bien l'alvérine sous forme ionique que sous forme de sels.

Préférentiellement selon l'invention, le sels organique est du citrate d'alvérine et le sel inorganique est le chlorure d'alvérine.

Cette utilisation s'avère particulièrement efficace pour diminuer les rides et ridules et/ou pour lisser la peau.

Ainsi, l'invention a également pour objet l'utilisation d'au moins une quantité efficace d'Alvérine ou de l'un de ses sels, dans une composition ou pour la préparation d'une composition, l'Alvérine ou son sel, ou la composition étant destinées à diminuer les rides et/ou les ridules.

Plus particulièrement la relaxation et/ou le relâchement du tissu cutané et/ou sous-cutané est un relâchement ou une relaxation musculaire.

L'utilisation de l'Alvérine selon l'invention peut être à titre préventif et/ou curatif.

L'invention a également pour objet une composition destinée à relaxer et/ou relâcher le tissu cutané et/ou sous-cutané comprenant au moins une quantité efficace d'Alvérine ou de l'un de ses sels.
Elle a également pour objet une composition destinée à diminuer les rides et ridules et/ou à lisser la peau comprenant au moins une quantité efficace d'Alvérine ou de l'un de ses sels.

Selon l'invention, la composition est destinée à un usage cosmétique ou dermatologique, de préférence cosmétique.

Selon l'invention, la composition comprenant de l'Alvérine ou de l'un de ses sels peut être appliquée soit par voie locale, c'est-à-dire par voie topique, ou par injection sous-cutanée et/ou intradermique, soit par voie systémique ou générale, c'est-à-dire par voie orale et/ou par injection intramusculaire.

Préférentiellement, la composition contenant l'Alvérine ou de l'un de ses sels est appliquée par voie topique.

La composition de l'invention, destinée à une application topique, contient un milieu physiologiquement acceptable, c'est-à-dire compatible avec la peau y compris le cuir chevelu, les muqueuses et/ou les yeux. La composition peut constituer notamment une composition cosmétique ou dermatologique.

L'invention a aussi pour objet un procédé de traitement cosmétique pour diminuer les rides et/ou les ridules, comprenant l'administration d'une composition comprenant au moins une quantité efficace d'Alvérine ou de l'un de ses sels.

Par administration, on entend selon l'invention l'application par voie topique, l'injection ou l'ingestion.

Préférentiellement selon l'invention, le procédé de traitement cosmétique comprend une application par voie topique.

La quantité d'Alvérine ou de l'un de ses sels utilisable selon l'invention dépend bien évidemment de l'effet recherché.

A titre d'exemple, la quantité pondérale d'Alvérine ou de l'un de ses sels utilisable selon l'invention peut par exemple être comprise entre 0,0001 % et 20 % et de préférence entre 0,001 % et 5 % du poids total de la composition.

Les compositions selon l'invention peuvent se présenter sous toutes les formes galéniques normalement utilisées pour une application topique, en injection ou par voie orale.

De préférence les compositions selon l'invention se présentent sous une forme galénique utilisée pour une application topique.

Les quantités des différents constituants des compositions selon l'invention sont celles classiquement utilisées dans les domaines considérés et sont appropriées à leur forme galénique.
Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de micro-émulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de traitement ou de soin cosmétique ou dermatologique pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition de l'invention est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou dermatologique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 50 % en poids, et de préférence de 0,5 à 30 % en poids par rapport au poids total de la composition.

De façon connue, les compositions cosmétiques ou dermatologiques de l'invention peuvent contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou dermatologique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions de l'invention, on peut notamment citer les autres actifs ayant un effet sur le traitement des rides ou des ridules, et en particulier les actifs kératolytiques. Par kératolytique, on entend un actif ayant des propriétés desquamantes, exfoliantes ou gommantes, ou un actif capable de ramollir la couche cornée.

Parmi les actifs ayant un effet sur le traitement des rides ou des ridules que peuvent contenir les compositions de l'invention, on peut en particulier citer les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001% à 5% en poids par rapport au poids total de la composition. Quand les compositions de l'invention sont destinées à une application par voie orale, elles peuvent se présenter sous les formes galéniques habituelles dans ce domaine, telles que les comprimés, les gélules, les produits buvables, notamment constitués extemporanément, les granulés, les poudres, dans les excipients habituels pour une telle application.

Quand les compositions de l'invention sont destinées à être injectées, elles peuvent se présenter sous forme de solutions contenant les excipients habituellement utilisés pour les injections, et par exemple sous forme d'une solution isotonique de chlorure de sodium.

Les exemples et compositions suivants illustrent l'invention sans la limiter aucunement. Dans les compositions les proportions indiquées sont des pourcentages en poids, sauf mention contraire.

Exemple 1 : Activité de l'Alvérine dans un modèle de jonction nerf / muscle (plaque motrice) mesurée dans une préparation nerf phrénique / diaphragme isolé de rat (muscle strié) (Pollard B.J. et al, Br.J. Anaesth., 1988, 61, 419-424).

Le nerf phrénique et le diaphragme sont soigneusement isolés et placés dans une cuve de 50 ml remplie de liquide de survie (liquide de Krebs Henseleit) maintenu à une température de 37°C et oxygéné à l'aide d'un mélange oxygène/CO₂ (95/5).

Les variations de tension du diaphragme sont enregistrées avec une pré charge initiale de plusieurs grammes.

Après une période de relaxation de 30 mn, le diaphragme est stimulé indirectement par l'intermédiaire du nerf phrénique.

Sur chaque préparation, l'effet des produits à tester a été dans un premier temps, évalué sur les contractions induites par stimulation indirecte via stimulation sur le nerf phrénique (0.1 à 0.5 volts, 0.3 ms, 0.1 Hz) aux concentrations croissantes et cumulées de 10⁻⁹ M à 10⁻⁴ M.

En cas d'effet à la fin du test, le diaphragme est stimulé directement afin de déterminer si l'inhibition de la neurotransmission résulte d'un blocage pré-jonctionnel ou d'un blocage post-jonctionnel.
Les résultats obtenus dans le modèle de plaque motrice avec l'Alvérine sont présentés dans le tableau ci-dessous :

| | Alverine 10⁻⁴ M | |
|---|---|---|
| % d'inhibition | 1ère étude | 2ème étude |
| stimulation indirecte | 100 | 100 |
| stimulation directe | 61.4 | 42.2 |

A la concentration de 10⁻⁴M, l'Alvérine inhibe complètement les contractions induites par les stimulations indirectes et inhibe dans une proportion d'environ 50% celles induites par les stimulations directes. En conclusion, l'Alvérine intervient préférentiellement sur la jonction neuromusculaire en faisant jouer les échanges calciques ce qui explique également son effet par stimulation direct.

### Exemple 2 : exemples de compositions selon l'invention.

| Composition 1 : Lotion de soin pour le visage | |
|---|---|
| Alvérine | 1,00 % |
| Antioxydant | 0,05 % |
| Conservateur | 0,30 % |
| Ethanol (solvant) | 8,00 % |
| Eau | qsp 100 % |

La lotion obtenue agit sur les rides lors d'une utilisation répétée (application biquotidienne pendant un mois).

| Composition 2 : Gel pour le soin du visage | |
|---|---|
| Alvérine | 2,00 % |
| Hydroxypropylcellulose * | 1,00 % |
| Conservateur | 0,30 % |
| Ethanol (solvant) | 15,00 % |
| Antioxydant | 0,05 % |
| Eau | qsp 100 % |

| | |
|---|---|
| * : Klucel H^{®} vendu par la société Hercules (gélifiant). | |

Le gel obtenu agit sur les rides. Il peut être appliqué quotidiennement matin et soir pendant un mois.

| Composition 3 : Crème de soin du visage (émulsion huile-dans-eau) | |
|---|---|
| Alvérine | 0,50 % |
| Stéarate de glycérol | 2,00 % |
| Polysorbate 60** | 1,00 % |
| Acide stéarique | 1,40 % |
| Triéthanolamine | 0,70 % |
| Carbomer*** | 0,40 % |
| Fraction liquide de beurre de karité | 12,00 % |
| Perhydrosqualène | 12,00 % |
| Conservateur | 0,30 % |
| Parfum | 0,50 % |
| Antioxydant | 0,05 % |
| Eau | qsp 100 % |

| | |
|---|---|
| ** : Tween 60® vendu par la société ICI ; | |
| *** : Carbopol 940® vendu par la société Goodrich) | |

On obtient une crème blanche, onctueuse, qui agit sur les rides et les ridules, et que l'on peut appliquer quotidiennement.

| Composition 4 : Crème de soin du visage (émulsion huile-dans-eau) | |
|---|---|
| Alvérine | 5,00 % |
| Mono-, distéarate de glycérol | 2,00 % |
| Alcool cétylique | 1,50 % |
| Mélange alcool cétylstéarylique/alcool cétylstéarylique oxyéthyléné 33 OE | 7,00 % |
| Diméthylpolysiloxane | 1,50 % |
| Huile de vaseline | 17,50 % |
| Conservateur | 0,30 % |
| Parfum | 0,50 % |
| Glycérine | 12,50 % |
| Eau | qsp 100 % |

## Revendications

1. Utilisation d'au moins une quantité efficace d'Alvérine ou de l'un de ses sels dans une composition ou pour la préparation d'une composition, l'Alvérine ou son sel, ou la composition étant destinées à relaxer et/ou relâcher le tissu cutané et/ou sous-cutané.

2. Utilisation d'au moins une quantité efficace d'Alvérine ou de l'un de ses sels dans une composition ou pour la préparation d'une composition, l'Alvérine ou son sel, ou la composition étant destinées à diminuer les rides et/ou les ridules.

3. Utilisation d'au moins une quantité efficace d'Alvérine ou de l'un de ses sels dans une composition ou pour la préparation d'une composition, l'Alvérine ou son sel, ou la composition étant destinées à lisser la peau.

4. Utilisation selon l'une quelconque des revendications précédentes, caractérisée en ce que l'Alvérine ou de l'un de ses sels est utilisée en une quantité pondérale comprise entre 0,0001 % et 10 % du poids total de la composition.

5. Utilisation selon l'une quelconque des revendications précédentes, caractérisée en ce que l'Alvérine ou de l'un de ses sels est utilisée en une quantité pondérale comprise entre 0,001 % et 5 % du poids total de la composition.

6. Utilisation selon l'une quelconque des revendications précédentes, caractérisée en ce que le sel d'alvérine est choisi parmi les sels organiques ou inorganiques.

7. Utilisation selon l'une quelconque des revendications précédentes, caractérisée en ce que le sel organique d'alvérine est choisi parmi le gluconate d'alvérine, l'acétate d'alvérine, le citrate d'alvérine, l'oléate d'alvérine ou l'oxalate d'alvérine.

8. Utilisation selon la revendication précédente, caractérisée en ce que le sel organique d'alvérine est le citrate d'alvérine.

9. Utilisation selon l'une quelconque des revendications 1 à 6, caractérisée en ce que le sel inorganique d'alvérine est choisi parmi le chlorure d'alvérine ou le borate d'alvérine ou le nitrate d'alvérine ou le phosphate d'alvérine ou le sulfate d'alvérine ou le carbonate d'alvérine.

10. Utilisation selon la revendication précédente, caractérisée en ce que le sel inorganique d'alvérine est le chlorure d'alvérine.

11. Composition destinée à relaxer et/ou relâcher le tissu cutané et/ou sous-cutané comprenant au moins une quantité efficace d'Alvérine ou de l'un de ses sels.

12. Composition destinée à diminuer les rides et ridules comprenant au moins une quantité efficace d'Alvérine ou de l'un de ses sels.

13. Composition destinée à lisser la peau comprenant au moins une quantité efficace d'Alvérine ou de l'un de ses sels.

14. Composition selon l'une quelconque des revendications 11 à 13, caractérisée en ce que le sel d'alvérine est choisi parmi les sels organiques ou inorganiques.

15. Composition selon l'une quelconque des revendications 11 à 14, caractérisée en ce que le sel organique d'alvérine est choisi parmi le gluconate d'alvérine, l'acétate d'alvérine, le citrate d'alvérine, l'oléate d'alvérine ou l'oxalate d'alvérine.

16. Composition selon la revendication précédente, caractérisée en ce que le sel organique d'alvérine est le citrate d'alvérine.

17. Composition selon l'une quelconque des revendications 11 à 14, caractérisée en ce que le sel inorganique d'alvérine est choisi parmi le chlorure d'alvérine ou le borate d'alvérine ou le nitrate d'alvérine ou le phosphate d'alvérine ou le sulfate d'alvérine ou le carbonate d'alvérine.

18. Composition selon la revendication précédente, caractérisée en ce que le sel inorganique d'alvérine est le chlorure d'alvérine.

19. Procédé de traitement cosmétique pour diminuer les rides et/ou les ridules, comprenant l'administration d'une composition comprenant au moins une quantité efficace d'Alvérine ou de l'un de ses sels.
